(19) Europäisches Patentamt
European Patent Office
Office européen des brevets

(11) Numéro de publication: **0 331 915 B1**

(12) # FASCICULE DE BREVET EUROPEEN

(45) Date de publication de fascicule du brevet: **28.07.93**

(51) Int. Cl.$^5$: **A61K 7/06**, A61K 7/48, A61K 7/00

(21) Numéro de dépôt: **89102113.1**

(22) Date de dépôt: **08.02.89**

(54) **Application cosmétique de polysiloxanes à fonction beta-cétoester.**

(30) Priorité: **08.02.88 LU 87127**

(43) Date de publication de la demande:
**13.09.89 Bulletin 89/37**

(45) Mention de la délivrance du brevet:
**28.07.93 Bulletin 93/30**

(84) Etats contractants désignés:
**AT BE CH DE FR GB GR IT LI NL SE**

(56) Documents cités:
**EP-A- 0 186 438**
**EP-A- 0 251 679**
**FR-A- 2 602 776**

(73) Titulaire: **L'OREAL**
**14, Rue Royale**
**F-75008 Paris(FR)**

(72) Inventeur: **Grollier, Jean-Francois**
**16 bis Boulevard Morland**
**F-75004 Paris(FR)**
Inventeur: **Dubief, Claude**
**9, Rue Edmond Rostand**
**F-78150 Le Chesnay(FR)**

(74) Mandataire: **Casalonga, Axel et al**
**BUREAU D.A. CASALONGA - JOSSE Morassistrasse 8**
**W-8000 München 5 (DE)**

**EP 0 331 915 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet l'utilisation en cosmétique de diorganopolysiloxanes à fonction β-cétoester, aux compositions cosmétiques mises en oeuvre ainsi qu'au procédé de traitement cosmétique, en particulier des cheveux et de la peau.

On utilise déjà des huiles siliconées en cosmétique comme lubrifiant dans les compositions destinées au traitement et aux soins des cheveux et de la peau. Il s'agit essentiellement de polydiméthylsiloxanes.

La demanderesse a découvert, de façon suprenante, que l'utilisation d'huiles siliconées constituées de diorganopolysiloxanes à fonction β-cétoester permettait d'obtenir des cheveux brillants et doux tout en ayant un toucher non gras. Ces composés présentent également l'avantage de conférer à la peau de la douceur et un toucher non poisseux.

On appelle "traitement cosmétique" un traitement visant à obtenir sur les cheveux un ou plusieurs des résultats indiqués ci-dessus. Il en est de même pour le traitement cosmétique de la peau.

Un objet de l'invention est donc constitué par l'utilisation cosmétique des diorganosiloxanes à fonction β-cétoester.

L'invention a également pour objet un procédé de traitement cosmétique des cheveux ou de la peau mettant en oeuvre ces composés.

Un autre objet de l'invention est constitué par les compositions cosmétiques destinées au traitement de la peau ou des cheveux, mettant en oeuvre ces composés.

Les diorganopolysiloxanes à fonction β-cétoester peuvent être utilisés comme excipients dans des compositions dermatologiques à usage topique contenant une substance active.

D'autres objets de l'invention apparaîtront à la lecture de la description et des exemples qui suivent.

Les diorganopolysiloxanes à fonction β-cétoester utilisés, conformément à l'invention, en cosmétique sont essentiellement caractérisés par le fait qu'ils répondent à la formule :

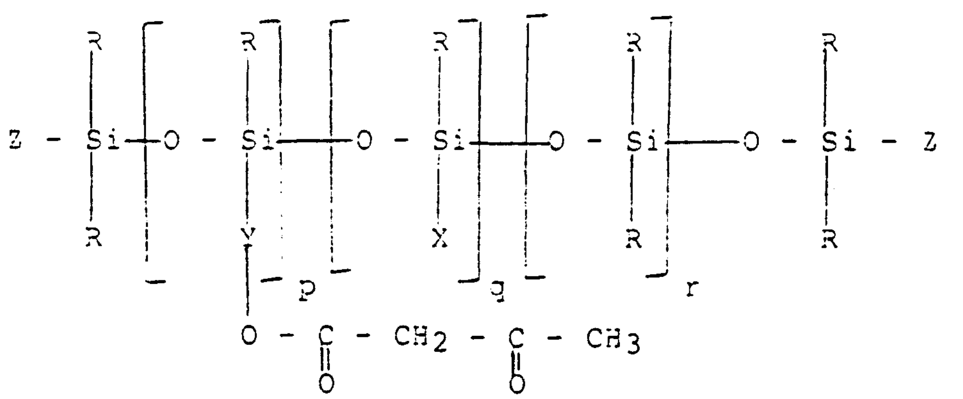

(I)

dans laquelle :

- les groupements R, identiques ou différents, désignent indépendamment l'un de l'autre, un groupement alkyl en $C_1$-$C_4$, trifluoro-3,3,3 propyle, vinyle, phényle, au moins 80% en nombre des radicaux R étant des radicaux méthyle,
- les groupements Y, identiques ou différents, désignent indépendamment l'un de l'autre, un chaînon alkylène, linéaire ou ramifié, en $C_1$-$C_{18}$, éventuellement prolongé par une chaîne polyéther choisie parmi le polyoxyde d'éthylène et le polyoxyde de propylène ou leurs mélanges,
- les groupements X, identiques ou différents, désignent indépendamment l'un de l'autre, un groupement Y-OH et Y-OCOR′, Y ayant la signification indiquée ci-dessus et R′ étant choisi parmi les groupements alkyle ou alcényle linéaires ou ramifiés en $C_1$-$C_{18}$,
- les groupements Z, identiques ou différents, désignent indépendamment l'un de l'autre, un radical R ayant la signification indiquée ci-dessus ou bien un radical

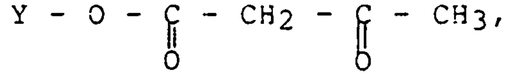

dans lequel Y a la signification indiquée ci-dessus, Z pouvant également désigner un groupement OR′

dans lequel R′ a la signification indiquée ci-dessus,

- p est un nombre entier compris entre 1 et 50 inclus et de préférence compris entre 1 et 16 inclus,
- q est un nombre entier compris entre 0 et 30 inclus et de préférence compris entre 0 et 8 inclus,
- r est un nombre entier compris entre 0 et 500 inclus et de préférence compris entre 2 et 50 inclus.

Lorsque les copolymères de formule (I) se présentent sous forme de mélange présentant les mêmes motifs mais en nombre différent, ce mélange peut être représenté par une formule (I) moyenne, dans laquelle p, q et r peuvent être des nombres décimaux.

Parmi les composés particulièrement préférés utilisés conformément à l'invention, on peut citer les composés de formule (I) dans laquelle :

R désigne un groupement alkyle choisi parmi les groupements méthyle, éthyle, propyle et butyle,

les motifs diorganosiloxanes $R_2SiO$ préférés sont les suivants :

$(CH_3)_2SiO$

$(CH_2=CH)(CH_3)SiO$

$(C_6H_5)(CH_3)SiO$

$(CF_3-CH_2-CH_2)(CH_3)SiO$

A titre de chaînon Y, on peut citer :

$-CH_2-$,$-(CH_2)_2-$,$-(CH_2)_3-(OCH_2-CH_2)_{29}-$,$-(CH_2)_3-$,

$-(CH_2)_3-[(O-CH_2-CH(CH_3)]_{15}-$,$-CH_2-CH(CH_3)-CH_2-$,

$-(CH_2)_{12}-$,

le groupement $-(CH_2)_3-$ est le groupement préféré pour Y,

Z désigne de préférence un radical R et plus particulièrement un groupement méthyle.

A titre de radical Y-OCOR′, on peut citer :

$CH_2 = CHCOOCH_2-$

$CH_2 = CHCOO(CH_2)_3-$

$C_8H_{15}COO(CH_2)_3-$

$CH_2 = C(CH_3)COO(CH_2)_3-$

Le radical R'COO représente de préférence en radical acrylate ou méthacrylate, c'est-à-dire que R' est un radical alcényle en $C_2$-$C_3$ inclus.

Les copolymères de formule (I) peuvent être des copolymères statistiques, à bloc ou séquencés, selon la nature des copolymères de départ utilisés pour la synthèse des copolymères de formule (I).

Les composés particulièrement préférés sont ceux dans lesquels R désigne un groupement méthyle; Z désigne méthyle et Y désigne un groupement

$-(CH_2)_3-$;

p est un nombre entier ou décimal compris entre 4,5 et 12; q est égal à O et r est un nombre entier ou décimal compris entre 6 et 45.

Les diorganopolysiloxanes à fonction $\beta$-cétoester répondant à la formule (I) sont connus et décrits dans la demande de brevet EP A 0263038.

Ils peuvent être préparés en faisant réagir une quantité appropriée de dicétène sur une huile hydroxylée, répondant à la formule :

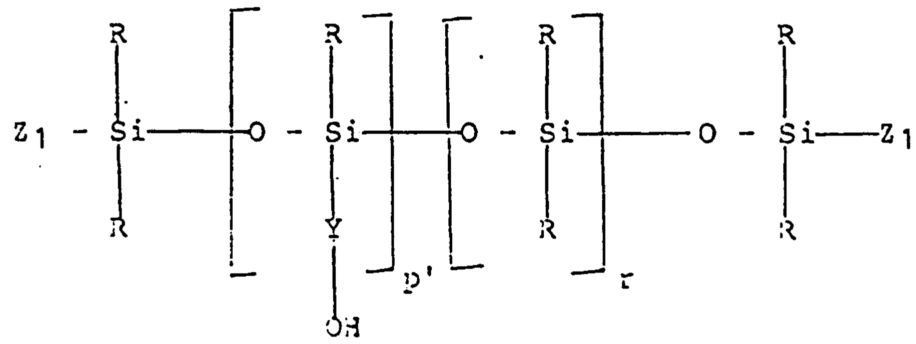 (II)

dans laquelle p' = p÷q, R, Y et r ont les significations indiquées en relation avec la formule (I) ci-dessus et $Z_1$ désigne un groupement R, ou bien Y-OH.

Cette réaction peut éventuellement être suivie dans le cas où q est différent de zéro d'une estérification des groupes hydroxyle résiduels.

La réaction peut s'effectuer en masse ou de préférence en milieu solvant organique tel que l'acétate d'éthyle à pression atmosphérique et de préférence en présence d'un catalyseur acide tel que l'acide p-toluène sulfonique ou basique tel qu'une amine tertiaire telle que la triméthylamine ou la triéthylamine.

La réaction d'un dicétène sur un groupe hydroxyalkyle est connue et a été décrite plus particulièrement par A.B. BOESE, Industrial and Engineering Chemistry, Int. Ed. 32, 1940, pages 16 à 25, et par R.N. LACEY "Advances in Organic Chemistry Methods and Results", Vol. 2, pages 240 à 248.

Dans le cas où l'on veut obtenir un polymère de formule (I) dans laquelle q est égal à 0 et p′ = p, on utilise un quantité stoechiométrique ou de préférence un excès de dicétène qui est éliminé en fin de réaction, par exemple par distillation sous pression réduite.

Dans le cas où l'on veut obtenir un polymère de formule (I) dans lequelle q est différent de 0, on utilise une quantité adaptée inférieure à la quantité stoechiométrique de dicétène en fonction des nombres p et q préalablement choisis.

Les compositions cosmétiques constituant l'un des objets de l'invention sont destinées au traitement et aux soins cosmétiques des cheveux et de la peau, et elles contiennent dans un milieu cosmétiquement acceptable, un diorganopolysiloxane à fonction $\beta$-cétoester répondant à la formule (I) définie ci-dessus dans des concentrations comprises de préférence entre 0,5 et 50% en poids par rapport au poids total de la composition et en particulier entre 1,5 et 30% en poids.

Ces compositions peuvent se présenter sous des formes diverses telles que des dispersions aqueuses, des lotions alcooliques ou hydroalcooliques, épaissies ou non et éventuellement conditionnées en aérosol.

Elles peuvent contenir en plus du diorganopolysiloxane de formule (I), des adjuvants habituellement utilisés en cosmétique tels que des parfums, des colorants, des conservateurs, des épaississants, des agents tensio-actifs anioniques, non ioniques ou amphotères ou leurs mélanges, des agents séquestrants, des stabilisateurs de mousse, des humectants, des filtres solaires, ainsi que des substances actives sur le plan cosmétique ou dermatologique.

Les compositions cosmétiques destinées au traitement des cheveux, conformes à l'invention, peuvent être utilisées en particulier comme shampooing, produits à rincer à appliquer avant ou après shampooing, avant ou après coloration ou décoloration, avant ou après permanente ou défrisage, comme produits coiffants non rincés tels que dans des lotions de mise en plis ou de brushing ou encore dans des laques.

Lorsque la composition constitue un shampooing, elle contient dans un milieu cosmétiquement acceptable, au moins un ou plusieurs agents de surface anioniques, non ioniques, amphotères ou leurs mélanges; la concentration totale en agents de surface étant généralement comprise entre 0,5 et 30% en poids par rapport au poids total de la composition, et de préférence entre 1,5 et 15% en poids.

Lorsque les compositions sont utilisées comme produits à rincer, ces produits peuvent se présenter sous forme de dispersions aqueuses ou lotions, éventuellement épaissies, ou de gels.

Les lotions peuvent contenir dans un milieu aqueux de 1 à 70% d'un solvant cosmétiquement acceptable et plus particulièrement choisi parmi les monoalcools inférieurs en $C_1$-$C_4$ tels que l'alcool éthylique, l'alcool n-propylique, l'alcool isopropylique, l'alcool tertiobutylique; les polyalcools tels que l'éthylèneglycol, le diéthylèneglycol, le propylèneglycol, des éthers de glycols tels que les mono- ou diéthylèneglycolalkyléthers; les esters d'acides gras tels que le myristate d'isopropyle.

Lorsque les compositions sont épaissies ou se présentent sous forme de gels, elles contiennent un ou plusieurs épaississants pouvant être choisis parmi l'alginate de sodium ou la gomme arabique, les dérivés

cellulosiques tels que la méthylcellulose, l'hydroxyméthylcellulose, l'hydroxyéthylcellulose, l'hydroxypropylcellulose, l'hydroxypropylméthylcellulose, la carboxyméthylcellulose, la gomme de guar ou ses dérivés, les hétérobiopolysaccharides tels que la gomme de xanthane ou les scléroglucanes, les polymères d'acide acrylique réticulés ou non. On peut également obtenir un épaississement des compositions par mélange de polyéthylèneglycol et de stéarate ou de distéarate de polyéthylèneglycol ou par un mélange d'esters phosphoriques et d'amides.

On peut épaissir la composition en utilisant le produit résultant de l'interaction ionique d'un polymère cationique constitué par un copolymère de cellulose ou d'un dérivé de cellulose greffé par un sel de monomère hydrosoluble d'ammonium quaternaire et d'un polymère anionique carboxylique ayant une viscosité capillaire absolue dans le diméthylformamide ou le méthanol à une concentration de 5% et à 30°C inférieure ou égale à $30 \times 10^{-3}$ Pa.s, tel que décrit plus particulièrement dans la demande de brevet français n° 2 598 611.

Les épaississants sont utilisés dans des concentrations comprises entre 0,1 et 30% en poids et de préférence entre 0,2 et 15% en poids par rapport au poids total de la composition.

Lorsque les compositions destinées au traitement des cheveux sont utilisées comme produits coiffants non rincés, elles contiennent dans un milieu aqueux ou solvant, les diorganopolysiloxanes de formule (I) en présence éventuellement d'agents épaississants tels que définis ci-dessus.

Les solvants sont choisis de préférence parmi les alcools inférieurs en $C_2$-$C_4$ et de préférence sont constitués par l'éthanol, et des silicones volatiles telles que les silicones cycliques connues dans le dictionnaire CTFA sous les dénominations HEXAMETHYLDISILOXANE et CYCLOMETHICONE, et leurs mélanges.

Les solvants sont utilisés dans des proportions comprises entre 5% et 99,5% en poids par rapport au poids total de la composition.

Les épaississants particulièrement préférés dans ce cas sont choisis parmi les polymères d'acide acrylique réticulés ou non et plus particulièrement les acides polyacryliques réticulés par un agent polyfonctionnel tels que les produits vendus sous la dénomination CARBOPOL par la Société GOODRICH, les dérivés de cellulose tels qu'indiqués ci-dessus, les copolymères éthylène/anhydride maléique tels que ceux vendus par la Société MONSANTO sous la dénomination EMA 91 et les copolymères de méthylvinyléther et d'anhydride maléique tels que ceux vendus par la Société GAF sous la dénomination GANTREZ AN (119, 139, 169).

La concentration en agents épaississants dans ces compositions varie entre 0,05 et 5% en poids et de préférence entre 0,1 et 2% en poids, par rapport au poids total de la composition.

Les compositions conformes à l'invention peuvent être conditionnées en aérosol pour être distribuées sous forme de sprays et former des laques. On utilise dans ce cas la composition en présence d'un gaz propulseur tel que plus particulièrement le gaz carbonique, l'azote, le protoxyde d'azote, l'éther diméthylique, les hydrocarbures volatils comme le butane, l'isobutane, le propane et les hydrocarbures chlorés et/ou fluorés et les mélanges d'hydrocarbures tels que le n-butane, l'isobutane, le propane, avec des hydrocarbures chlorofluorés.

Les compositions cosmétiques destinées au traitement ou aux soins de la peau peuvent être appliquées en particulier sous forme de produits pour le bain ou la douche, d'huiles corporelles, de produits bronzants, de produits pour le rasage, de lotions parfumées, de crèmes ou de laits.

Ces compositions contiennent dans un milieu cosmétiquement acceptable, approprié pour l'application sur la peau et bien connu de l'homme de métier, les dérivés de diorganopolysiloxanes de formule (I) définis ci-dessus, dans des proportions également définies.

Le procédé de traitement cosmétique mettant en oeuvre les diorganopolysiloxanes de formule (I) définis ci-dessus consiste essentiellement à appliquer la composition, soit sur les cheveux, suivant l'usage envisagé (shampooing, traitement à rincer, traitement de coiffage sans rinçage), sont sur la peau (bain, douche, etc...

Les exemples suivants sont destinés à illustrer l'invention.

Les exemples de référence ci-après sont destinés à illustrer la préparation d'un certain nombre de composés conformes à l'invention, alors que les exemples de compositions illustrent l'application cosmétique de ces composés.

EXEMPLE DE REFERENCE 1

Dans un ballon tricol de 500 ml muni d'une agitation centrale, d'un réfrigérant ascendant et d'une gaine thermométrique, on charge 100 g d'une huile hydroxypropylée de formule moyenne :

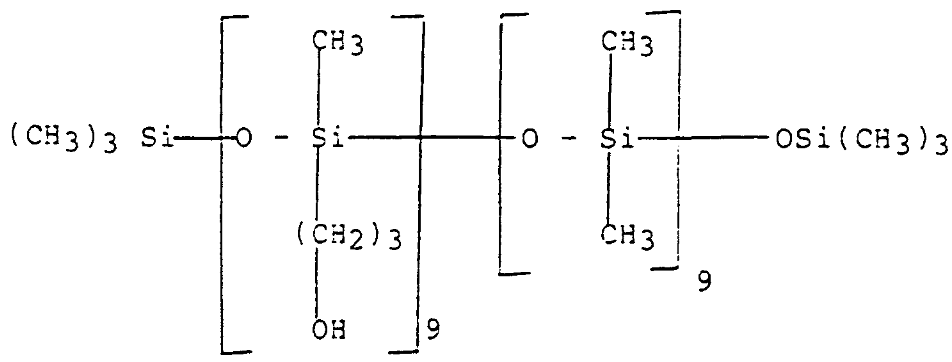

titrant 468 meq/100 g en fonction alcool. On ajoute 1 ml de triéthylamine comme catalyseur et 150 ml d'un solvant qui est l'acétate d'éthyle, puis on chauffe le mélange homogène ainsi obtenu à 45-50 ° C. On coule alors, en une heure, 41,5 g (0,494 mole) de dicétène en maintenant la température à 50 ° C. On note une légère exothermicité, notamment en début de coulée. En fin de coulée, on laisse encore une heure à 50 ° C, puis on laisse revenir le mélange réactionnel à température ambiante. On élimine l'excès de dicétène par évaporation sous pression réduite (0,7 Kpa) à 50-60 ° C. On obtient alors une huile jaune trouble que l'on filtre pour obtenir 138 g d'une huile jaune, limpide et inodore.

Par acidimétrie au moyen de soude aqueuse 0,5 N en milieu eau/acétone, on vérifie l'absence d'acidité forte (acide déhydroacétique) et on titre une acidité faible (de pKa environ 10) de 331 meq/100 g et représentant la fonction $\beta$-cétoester. Le rendement en fonction $\beta$-cétoester par rapport au nombre de fonction alcool engagé est de 97,6%.

Le produit obtenu est représenté par la formule moyenne suivante :

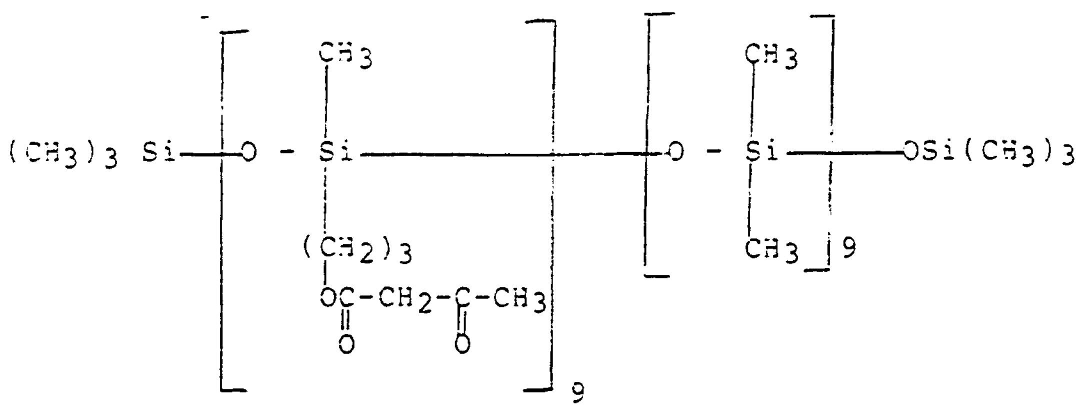

EXEMPLES DE REFERENCE 2 A 6

En opérant comme à l'exemple 1 mais en modifiant la composition de l'huile gamma-hydroxypropylée initiale, on prépare les produits suivants de formule moyenne :

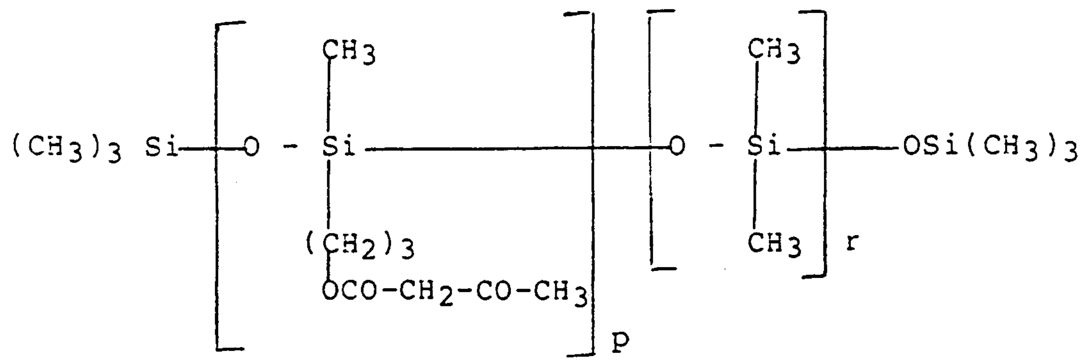

Les valeurs de p et r ainsi que le titre t en fonction β-cétoester exprimé en meq/100 g sont rassemblés dans le tableau I ci-après.

TABLEAU 1

| Ex | p | r | t |
|---|---|---|---|
| 2 | 4,5 | 11 | 230 |
| 3 | 12 | 17 | 298 |
| 4 | 12 | 45 | 197 |
| 5 | 12 | 6 | 362 |
| 6 | 9 | 7,5 | 330 |

## EXEMPLE DE REFERENCE 7

On reprend le mode opératoire de l'exemple 1, en partant de la même huile hydroxypropylée (100 g, 468 meq/100 g en fonction alcool), sauf que l'on coule une quantité moindre de dicétène (20 g, soit 0,238 mole).

On obient une huile jaune, limpide, inodore de formule moyenne :

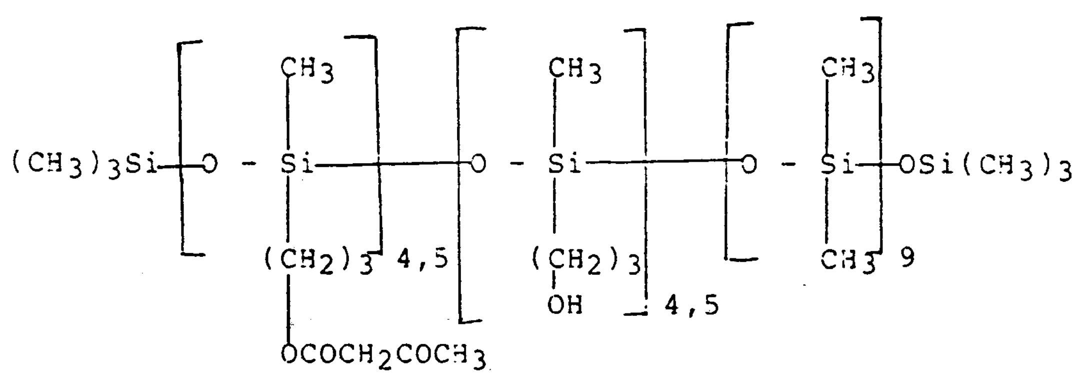

et présentant un titre en fonction acétoacétate de 188 meq/100 g.

EXEMPLE DE REFERENCE 8

On part d'une huile gamma-hydroxypropylée de formule moyenne :

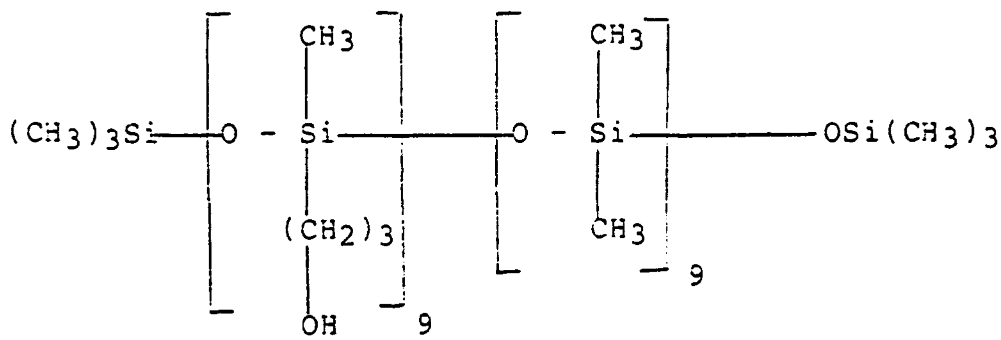

Sur cette huile on effectue une estérification partielle des fonctions alcool par du méthacrylate de méthyle en présence d'oxyde de dibutylétain.

On obtient une huile de formule :

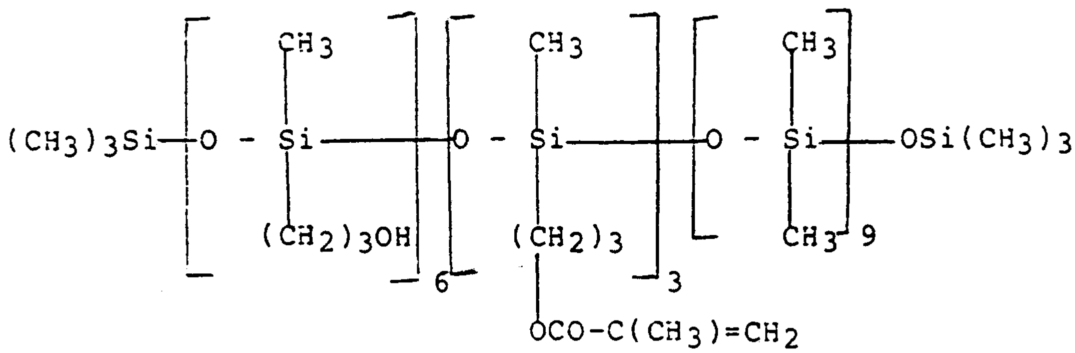

On fait réagir le dicétène en léger excès en opérant comme à l'exemple 1. On obtient après élimination de l'excès de dicétène une huile jaune, limpide et inodore de formule approchée :

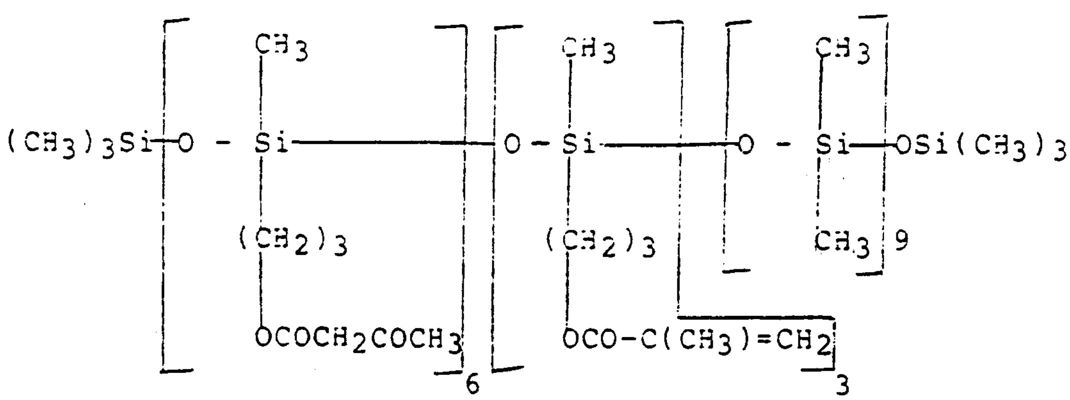

titrant 220 meq/100 g en fonction acétoacétate.

EXEMPLE DE COMPOSITION 1

On prépare un gel de coiffage à rincer de composition suivante :

- Diorganopolysiloxane à fonction ß-cétoester de formule : 15 g

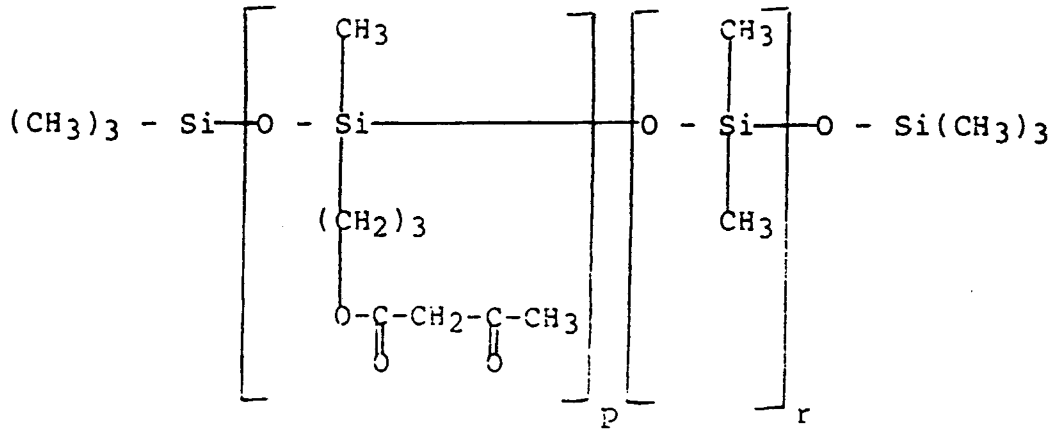

p = 9 r = 7,5

Titre en fonction ß-cétoester en meq/100 g = 330

- Acide polyacrylique réticulé PM = 4 millions, vendu sous la dénomination CARBOPOL 940 par la Société GOODRICH (neutralisé par $NH_4OH$) 1 g
- Eau qsp 100 g

On applique ce gel sur des cheveux lavés et essorés. Après rinçage, les cheveux sont doux et les cheveux séchés sont brillants.

EXEMPLE DE COMPOSITION 2

On prépare un spray à appliquer sur des cheveux séchés de composition suivante :

- Diorganopolysiloxane à fonction β-cétoester de formule : 2 g

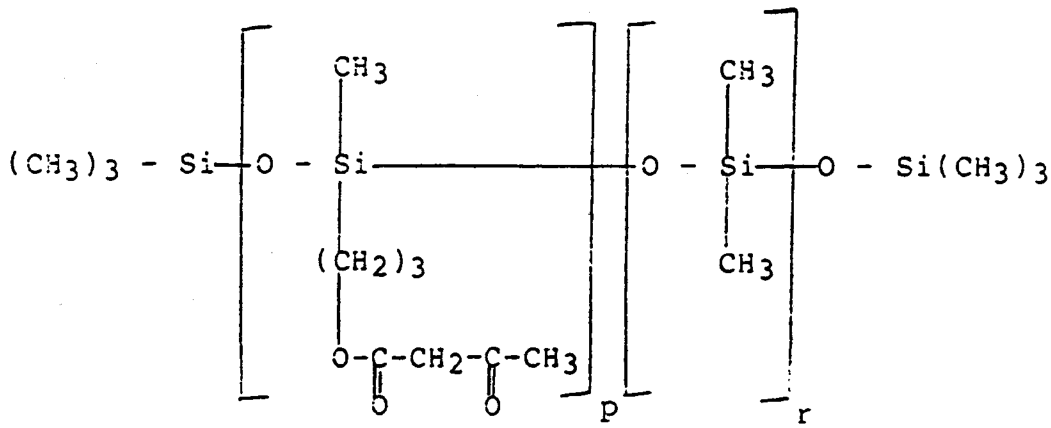

p = 9 r = 7,5

Titre en fonction β-cétoester en meq/100 g = 330

- Alcool éthylique absolu qsp 100 g

La composition est conditionnée en flacon pompe. Le spray apporte du brillant aux cheveux sans les graisser.

EXEMPLE DE COMPOSITION 3

On prépare un gel de coiffage à appliquer sur des cheveux séchés de composition suivante :

- Diorganopolysiloxane à fonction ß-cétoester de formule : 20 g

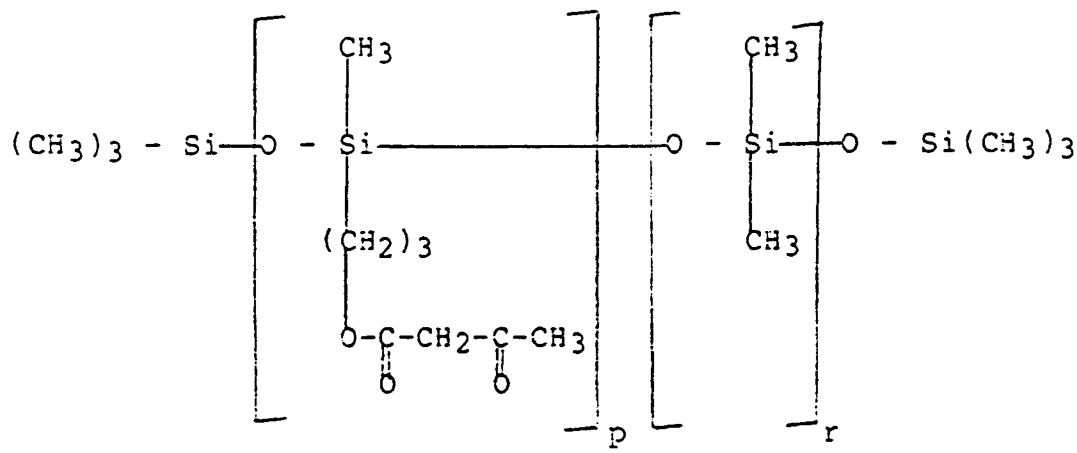

p = 9 r = 7,5

Titre en fonction ß-cétoester en meq/100 g = 330

- Acide polyacrylique réticulé PM = 4 millions, vendu sous la dénomination CARBOPOL 940 par la Société GOODRICH (neutralisé par NaOH) 1 g
- Parfum qs
- Eau qsp 100 g

Avec une application de ce gel, les cheveux sont doux et deviennent brillants.

EXEMPLE DE COMPOSITION 4

On prépare un gel douche de composition suivante :

| | |
|---|---|
| - Alkyl($C_{12}$-$C_{14}$)éther sulfate de sodium oxyéthylèné à 2,2 moles d'oxyde d'éthylène en solution aqueuse à 25% MA | 10,0 g MA |
| - Composé zwitterionique dérivé de la cocoamidopropyldiméthylglycine, vendu à 30% MA en solution aqueuse sous la dénomination TEGO BETAINE HS par la Société GOLDSCHMIDT | 8,0 g MA |
| - Isoparaffine vendue par la Société EXXON CHEMICALS sous la dénomination ISOPAR H | 5,0 g |
| - Diorganopolysiloxane à fonction ß-cétoester de formule : | 1,0 g |

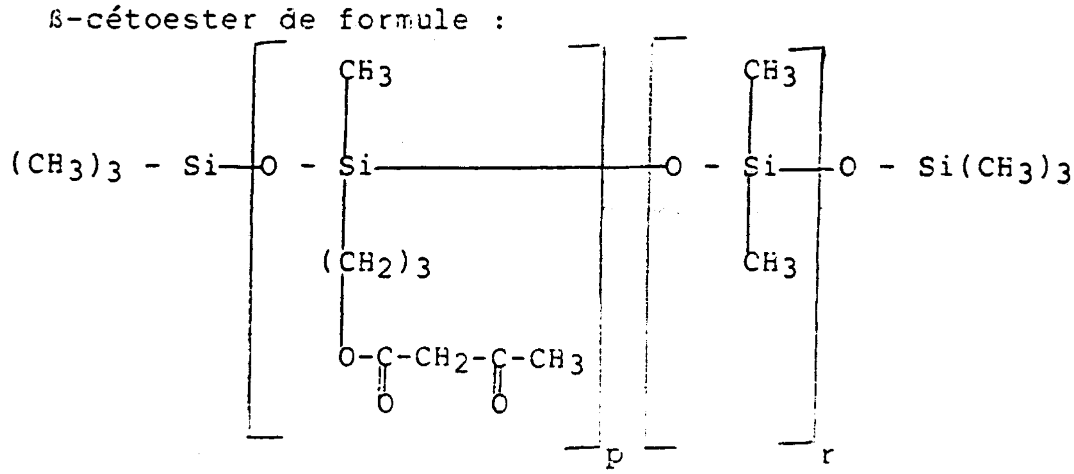

p = 9 r = 7,5

Titre en fonction ß-cétoester en meq/100 g = 330

| | |
|---|---|
| - Eau | qsp 100,0 g |
| - HCl qs pH = 5,5 | |

Ce gel douche au bon pouvoir moussant confère à la peau un toucher doux et non poisseux.

EXEMPLE DE COMPOSITION 5

On prépare une crème adoucissante pour le corps de compositions suivante :

| | |
|---|---|
| - Alcool cétylique | 3,0 g |
| - Mélange d'alcool cétylstéarylique et d'alcool cétylstéarylique oxyéthyléné à 33 moles d'oxyde d'éthylène, vendu sous la dénomination SINNOWAX AO par la Société HENKEL | 2,0 g |
| - Stéarate de diéthylèneglycol | 2,0 g |
| - Huile de vaseline | 10,0 g |
| - Gomme de xanthane vendue sous la dénomination KELTROL T par la Société KELCO | 0,5 g |
| - Diorganopolysiloxane à fonction ß-cétoester de formule : | 2,0 g |

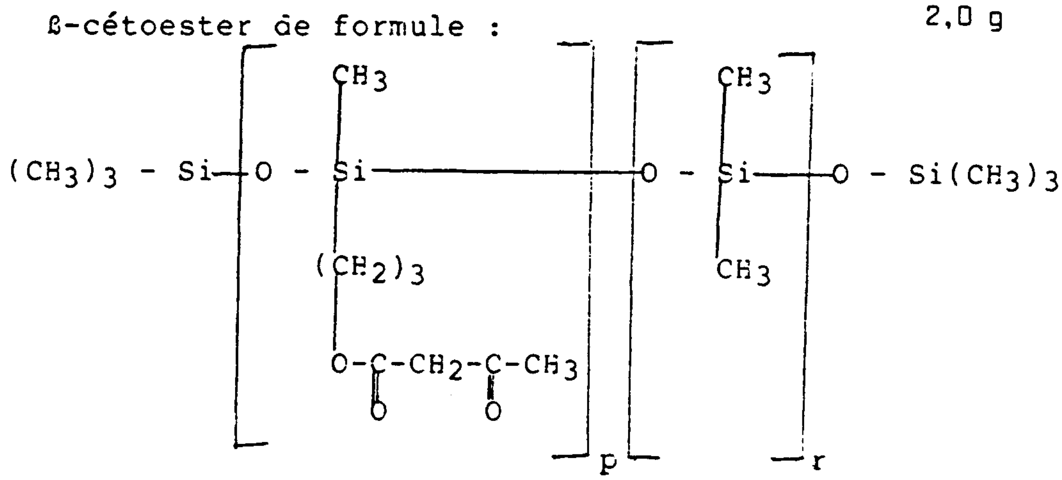

p = 9          r = 7,5

Titre en fonction ß-cétoester en meq/100 g = 330

| | | |
|---|---|---|
| - Eau | qsp | 100,0 g |
| - Triéthanolamine | qs | pH = 6 |

Cette crème d'application aisée apporte douceur et souplesse à la peau.

## Revendications

**1.** Utilisation en cosmétique de diorganopolysiloxanes à fonction β-cétoester, répondant à la formule :

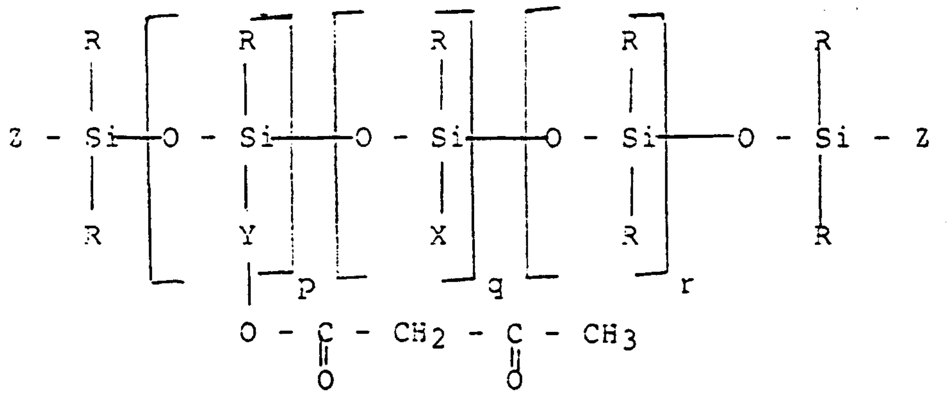

(I)

dans laquelle :

- les groupements R, identiques ou différents, désignent indépendamment l'un de l'autre, des groupements alkyle en $C_1$-$C_4$, trifluoro-3,3,3-propyle,vinyle, phényle, au moins 80% en nombre des groupements R étant des radicaux méthyle;
- les groupements Y, identiques ou différents, désignent indépendamment l'un de l'autre, un chaînon alkylène, linéaire ou ramifié en $C_1$-$C_{18}$, éventuellement prolongé par un chaînon polyéther;
- les groupements X, identiques ou différents, désignent indépendamment l'un de l'autre, un radical Y-OH ou Y-OCOR′, Y ayant la signification indiquée ci-dessus et R′ étant choisi parmi les groupements alkyle ou alcényle, linéaires ou ramifiés en $C_1$-$C_{18}$;
- les groupements Z, identiques ou différents, désignent indépendamment l'un de l'autre, un groupement R tel que défini ci-dessus ou bien un radical

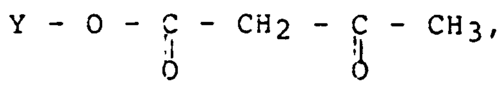

  Y ayant la signification indiqué ci-dessus, Z pouvant également désigner un groupement OR′ dans lequel R′ a la signification indiquée ci-dessus;
- p est un nombre entier compris entre 1 et 50 inclus et de préférence entre 1 et 16 inclus;
- q est un nombre entier compris entre 0 et 30 inclus et de préférence entre 0 et 8 inclus;
- r est un nombre entier compris entre 0 et 500 inclus et de préférence entre 2 et 50 inclus; ou un mélange de ces copolymères de formule (I), auquel cas, p, q et r peuvent être des nombres décimaux.

**2.** Utilisation selon la revendication 1, caractérisée par le fait que dans le composé de formule (I) le groupement R désigne un groupement méthyle, éthyle, propyle ou butyle; que les groupements diorganosiloxanes de formule $R_2SiO$ sont choisis parmi :

$(CH_3)_2SiO$
$(CH_2 = CH)(CH_3)SiO$
$(C_6H_5)(CH_3)SiO$
$(CF_3\text{-}CH_2\text{-}CH_2)(CH_3)SiO$ ;

que les chaînons Y sont choisis parmi :

$-CH_2-$,$-(CH_2)_2-$,$-(CH_2)_3-(OCH_2-CH_2)_{29}-$,$-(CH_2)_3-$,
$-(CH_2)_3-[(O-CH_2-CH(CH_3)]_{15}-$,$-CH_2-CH(CH_3)-CH_2-$,
$-(CH_2)_{12}-$ ;

que Z désigne un radical méthyle ;

que le radical Y-OCOR′ désigne un groupement choisi parmi :

$CH_2 = CHCOOCH_2-$

$$CH_2 = CHCOO(CH_2)_3-$$
$$C_8H_{15}COO(CH_2)_3-$$
$$CH_2 = C(CH_3)COO(CH_2)_3- \;;$$

que le radical R'COO représente un radical acrylate ou méthacrylate.

**3.** Composition de traitement cosmétique des cheveux ou de la peau, caractérisée par le fait qu'elle contient dans un milieu cosmétiquement acceptable un diorganopolysiloxane à fonction $\beta$-cétoester de formule (I) tel que défini dans l'une quelconque des revendications 1 ou 2, dans des concentrations comprises entre 0,5 et 50% en poids par rapport au poids total de la composition.

**4.** Composition selon la revendication 3, caractérisée par le fait qu'elle se présente sous forme de dispersions aqueuses, de lotions alcooliques ou hydroalcooliques, épaissies ou non, éventuellement conditionnées en aérosol.

**5.** Composition selon la revendication 3 ou 4, caractérisée par le fait qu'elle contient au moins un ou plusieurs adjuvants habituellement utilisé(s) en cosmétique, choisis parmi les parfums, les colorants, des conservateurs, les épaississants, les agents tensio-actifs anioniques, non ioniques ou amphotères ou leurs mélanges, les séquestrants, les stabilisateurs de mousse, les humectants, les filtres solaires, les substances actives en cosmétique et en dermatologie.

**6.** Composition selon l'une quelconque des revendications 3 à 5, destinée à être utilisée comme shampooing, caractérisée par le fait qu'elle utilisée comme plus au moins un ou plusieurs agents de surface anioniques, non ioniques, amphotères ou leurs mélanges, dans des proportions comprises entre 0,5 et 30% par rapport au poids total de la composition.

**7.** Composition selon l'une quelconque des revendications 3 à 5, destinée à être utilisée comme produit à rincer, caractérisée par le fait qu'elle se présente sous forme de dispersion aqueuse, de lotion, de lotion épaissie ou de gel.

**8.** Composition selon la revendication 7, caractérisée par le fait qu'elle contient 1 à 70% de solvant choisi parmi les alcanols inférieurs, les polyalcools, les éthers de glycols et les esters d'acides gras.

**9.** Composition selon l'une quelconque des revendications 6 à 8, caractérisée par le fait qu'elle est épaissie ou gélifiée avec un agent choisi parmi l'alginate de sodium, la gomme arabique, les dérivés cellulosiques, la gomme de guar, les hétérobiopolysaccharides, les polymères d'acide acrylique réticulés ou non, un mélange de polyéthylèneglycol et de stéarate ou de distéarate de polyéthylèneglycol, un mélange d'ester phosphorique et d'amide, le produit d'interaction ionique entre un polymère cationique constitué par un copolymère de cellulose ou un dérivé de cellulose greffés par un sel de monomère hydrosoluble d'ammonium quaternaire et un polymère anionique carboxylique ayant une viscosité capillaire absolue, dans le diméthylformamide ou le méthanol à une concentration de 5% et à 30 °C inférieure ou égale à $30 \times 10^{-3}$ Pa.s, cet agent épaississant est présent dans des proportions comprises entre 0,1 et 30% en poids par rapport au poids total de la composition.

**10.** Composition selon l'une quelconque des revendications 3 à 5, destinée à être utilisée comme produit coiffant, sans mettre en oeuvre de rinçage, caractérisée par le fait qu'elle contient en milieu aqueux ou solvant les diorganopolysiloxanes à fonction $\beta$-cétoester tels que définis dans la revendication 1 ou 2, les solvants étant choisis parmi les alcanols inférieurs en $C_2$-$C_4$ et les silicones volatiles.

**11.** Composition selon la revendication 10, caractérisée par le fait qu'elle contient les épaississants choisis parmi les polymères d'acide acrylique réticulés ou non, les dérivés de cellulose, les copolymères éthylène/anhydride maléique, les copolymères de méthylvinyléther et d'anhydride maléique, dans des proportions comprises entre 0,05 et 5% en poids.

**12.** Composition selon l'une quelconque des revendications 3 à 11, caractérisée par le fait qu'elle est conditionnée en aérosol pour former au moment de l'expulsion un spray, en présence d'un gaz propulseur.

**13.** Utilisation de la composition telle que définie dans l'une quelconque des revendications 3 à 12, dans le traitement cosmétique des cheveux.

**14.** Utilisation de la composition selon l'une quelconque des revendications 3 à 5, comme produit de bain ou de douche, d'huile corporelle, de produit bronzant, de produit pour le rasage, de lotion parfumée, de crème ou de lait.

**15.** Procédé de traitement cosmétique des cheveux en vue d'améliorer le brillant, la douceur, sans les graisser, caractérisé par le fait que l'on applique sur ceux-ci au moins une composition telle que définie dans l'une quelconque des revendications 3 à 12.

**16.** Procédé de traitement cosmétique de la peau, caractérisé par le fait que l'on applique sur celle-ci au moins une composition telle que définie dans l'une quelconque des revendications 3 à 5.

**Claims**

**1.** Use in cosmetics of diorganopolysiloxanes containing a $\beta$-keto ester group, corresponding to the formula:

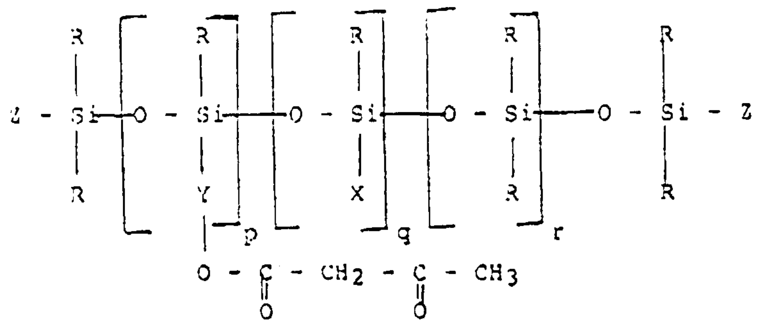

(I)

in which:

the groups R, which may be identical or different, denote, independently of one another, $C_1$-$C_4$ alkyl, 3,3,3-trifluoropropyl, vinyl or phenyl groups, at least 80%, in numerical terms of the groups R being methyl radicals;

the groups Y, which may be identical or different, denote, independently of one another, a $C_1$-$C_{18}$ linear or branched alkylene link, optionally extended by a polyether chain;

the groups X, which may be identical or different, denote, independently of one another, a radical Y-OH or Y-OCOR', Y having the meaning stated above and R' being chosen from $C_1$-$C_{18}$ linear or branched alkyl or alkenyl groups;

the groups Z, which may be identical or different, denote, independently of one another, a group R as defined above or alternatively a radical

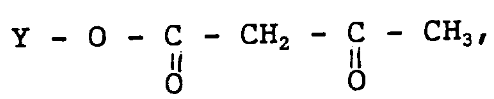

Y having the meaning stated above, it also being possible for Z to denote a group OR' in which R' has the meaning stated above;

p is an integer between 1 and 50 inclusive, and preferably between 1 and 16 inclusive;

q is an integer between 0 and 30 inclusive, and preferably between 0 and 8 inclusive; and

r is an integer between 0 and 500 inclusive, and preferably between 2 and 50 inclusive;

or a mixture of these copolymers of formula (I), in which case p, q and r can be decimal numbers.

**2.** Use according to Claim 1, characterized in that, in the compound of formula (I), the groups R denotes a methyl, ethyl, propyl or butyl group; in that the diorganosiloxane groups of formula $R_2SiO$ are chosen from;

$(CH_3)_2SiO$
$(CH_2=CH)(CH_3)SiO$
$(C_6H_5)(CH_3)SiO$
$(CF_3\text{-}CH_2\text{-}Ca_2)(CH_3)SiO$
in that the links Y are chosen from:
$-CH_2-$,$-(CH_2)_2-$, $-(CH_2)_3-(OCH_2-CH_2)_{29}-$, $-(CH_2)_3-$,
$-(CH_2)_3-[(O-CH_2-CH(CH_3)]_{15}-$, $-CH_2-CH(CH_3)-CH_2-$,
$-(CH_2)_{12}-$;
in that Z denotes a methyl radical;
in that the radical Y-OCOR' denotes a group chosen from: $CH_2 = CHCOOCH_2-$

$CH_2 = CHCOO(CH_2)_3-$
$C_8H_{15}COO(CH_2)_3-$
$CH_2 = C(CH_3)COO(CH_2)_3-$

and in that the radical R'COO denotes an acrylate or methacrylate radical.

**3.** Composition for cosmetic treatment of the hair or the skin, characterized in that it contains, in a cosmetically acceptable medium, a diorganopolysiloxane containing a $\beta$-keto ester group of formula (I) as defined in either of Claims 1 and 2, in concentrations of between 0.5 and 50% by weight relative to the total weight of the composition.

**4.** Composition according to Claim 3, characterized in that it is presented in the form of aqueous dispersions or alcoholic or aqueous-alcoholic lotions, thickened or otherwise, optionally packaged as an aerosol.

**5.** Composition according to Claim 3 or 4, characterized in that it contains at least one or more adjuvants customarily used in cosmetics, chosen from perfumes, colourings, preservatives, thickeners, anionic, nonionic or amphoteric surfactants or mixtures thereof, sequestering agents, foam stabilizers, humectants, sunscreens and substances which are active in cosmetics and in dermatology.

**6.** Composition according to any one of Claims 3 to 5, intended for use as a shampoo, characterized in that it contains, in addition, at least one or more anionic, nonionic or amphoteric surfactants or mixtures thereof, in proportions of between 0.5 and 30% by weight relative to the total weight of the composition.

**7.** Composition according to any one of Claims 3 to 5, intended for use as a product to be rinsed, characterized in that it is presented in the form of an aqueous dispersion, lotion, thickened lotion or gel.

**8.** Composition according to Claim 7, characterized in that it contains at least 1 to 70% of solvent, chosen from lower alkanols, polyhydric alcohols, glycol ethers and fatty acid esters.

**9.** Composition according to any one of Claims 6 to 8, characterized in that it is thickened or gelled with an agent chosen from sodium alginate, gum arabic, cellulose derivatives, guar gum, heterobiopolysaccharides, acrylic acid polymers, crosslinked or otherwise, a mixture of polyethylene glycol and polyethylene glycol stearate or distearate, a mixture of amide and phosphoric ester, and the product of ionic interaction between a cationic polymer comprising a copolymer of cellulose or a cellulose derivative, these being grafted with a water-soluble quaternary ammonium monomer salt, and a carboxylic anionic polymer having an absolute capillary viscosity in dimethylformamide or methanol at a concentration of 5% and at 30 °C of not more than $30 \times 10^{-3}$ Pa.s; this thickening agent is present in proportions of between 0.1 and 30% by weight relative to the total weight of the composition.

**10.** Composition according to any one of Claims 3 to 5, intended for use as a hair-styling product, without rinsing being employed, characterized in that it contains, in an aqueous or solvent medium, the diorganopolysiloxanes containing a $\beta$-keto ester group as defined in Claim 1 or 2, the solvents being chosen from $C_2$-$C_4$ lower alkanols and volatile silicones.

**11.** Composition according to Claim 10, characterized in that it contains thickeners chosen from acrylic acid polyaers, crosslinked or otherwise, cellulose derivatives, ethylene/maleic anhydride copolymers and copolymers of methyl vinyl ether and maleic anhydride, in proportions of between 0.05 and 5% by weight.

**12.** Composition according to any one of Claims 3 to 11, characterized in that it is packaged as an aerosol, to form a spray at the time of expulsion, in the presence of a propellant gas.

**13.** Use of the composition as defined in any one of Claims 3 to 12 in the cosmetic treatment of the hair.

**14.** Use of the composition according to any one of Claims 3 to 5 as a bath or shower product, body oil, tanning product, shaving product, perfumed lotion, cream or milk.

**15.** Process for cosmetic treatment of the hair for the purpose of improving the sheen and the softness without making it greasy, characterized in that at least one composition as defined in any one of Claims 3 to 12 is applied on the hair.

**16.** Process for cosmetic treatment of the skin, characterized in that at least one composition as defined in any one of Claims 3 to 5 is applied on the skin.

**Patentansprüche**

**1.** Verwendung auf kosmetischem Gebiet von Diorganopolysiloxanen mit $\beta$-Ketoesterfunktion, die der Formel entsprechen:

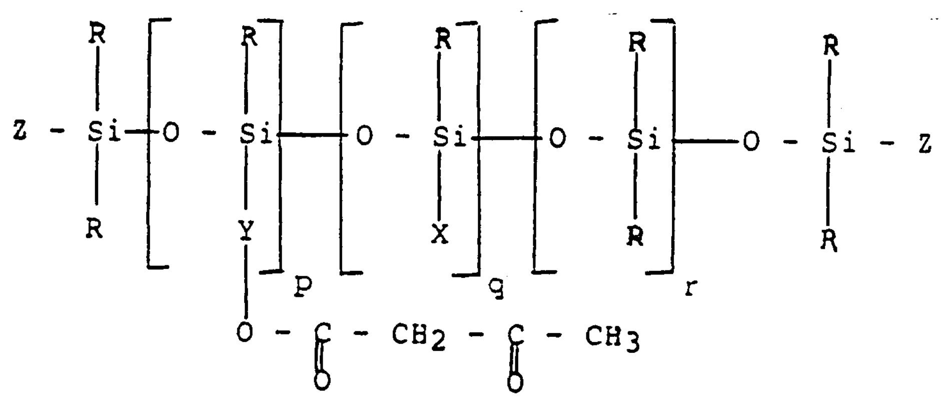

(I)

worin:

- die identischen oder Verschiedenartigen Gruppen R unabhängig voneinander $C_1$-$C_4$-Alkyl-, 3,3,3-Trifluorpropyl-, Vinyl-, Phenyl-gruppen bedeuten, wobei wenigstens 80 % der Anzahl der Gruppen R Methylreste sind;
- die identischen oder verschiedenartigen Gruppen Y unabhängig voneinander eine lineare oder verzweigte, gegebenenfalls durch eine Polyetherkette verlängerte, $C_1$-$C_{18}$-Alkylenkette bedeuten;
- die identischen oder verschiedenartigen Gruppen X unabhängig voneinander einen Rest Y-OH oder Y-OCOR', worin Y die zuvor angegebene Bedeutung hat und R' unter linearen oder verzweigten $C_1$-$C_{18}$-(Alkyl- oder Alkenylgruppen) ausgewählt ist, bedeuten;
- die identischen oder verschiedenartigen Gruppen Z unabhängig voneinander eine Gruppe R wie zuvor definiert oder auch einen Rest

$$Y - O - \underset{\underset{O}{\|}}{C} - CH_2 - \underset{\underset{O}{\|}}{C} - CH_3$$

bedeuten,
worin Y die zuvor angegebene Bedeutung hat, Z ebenfalls eine Gruppe OR', worin R' die zuvor angegebene Bedeutung hat, bedeuten kann;

- p eine ganze Zahl zwischen 1 und 50 einschließlich und vorzugsweise zwischen 1 und 16 einschließlich ist;
- q eine ganze Zahl zwischen 0 und 30 einschließlich und vorzugsweise zwischen 0 und 8 einschließlich ist;
- r eine ganze Zahl zwischen 0 und 500 einschließlich und vorzugsweise zwischen 2 und 50 einschließlich ist;

oder eine Mischung dieser Copolymeren der Formel (I), wobei in diesem Fall p, q und r Dezimalzahlen sein können.

**2.** Verwendung nach Anspruch 1, dadurch gekennzeichnet, daß in der Verbindung der Formel (I) die Gruppe R eine Methyl-, Ethyl-, Propyl- oder Butylgruppe bedeutet; daß die Diorganosiloxangruppen der Formel $R_2SiO$ ausgewählt sind unter:
$(CH_3)_2SiO$
$(CH_2=CH)(CH_3)SiO$
$(C_6H_5)(CH_3)SiO$
$(CF_3\text{-}CH_2\text{-}CH_2)(CH_3)SiO$;
daß die Kettenglieder Y ausgewählt sind aus:
$-CH_2-$, $-(CH_2)_2-$, $-(CH_2)_3-(OCH_2-CH_2)_{29}-$, $-(CH_2)_3-$,
$-(CH_2)_3-[(O-CH_2-CH(CH_3)]_{15}-$, $-(CH_2)-CH(CH_3)-CH_2-$,
$-(CH_2)_{12}-$;
daß Z einen Methylrest bezeichnet;
daß der Rest Y-OCOR' eine Gruppe bedeutet, ausgewählt aus:
$CH_2 = CHCOOCH_2-$
$CH_2 = CHCOO(CH_2)_3-$
$C_8H_{15}COO(CH_2)_3-$
$CH_2 = C(CH_3)COO(CH_2)_3-$;
daß der Rest R'COO einen Acrylat- oder Methacrylatrest darstellt.

**3.** Zusammensetzung für kosmetische Behandlung der Haare oder der Haut, dadurch gekennzeichnet, daß sie in einem kosmetisch annehmbaren Medium ein Diorganopolysiloxan mit $\beta$-Ketoesterfunktion der Formel (I), wie in einem der Ansprüche 1 oder 2 definiert, in Konzentrationen zwischen 0,5 und 50 Gew.-%, bezogen auf das Gesamtgewicht der Zusammensetzung, enthält.

**4.** Zusammensetzung nach Anspruch 3, dadurch gekennzeichnet, daß sie in Form von wässrigen Dispersionen, von alkoholischen oder wässrig-alkoholischen, verdickten oder nicht-verdickten Lotionen, gegebenenfalls als Aerosol konditioniert, vorliegt.

**5.** Zusammensetzung nach Anspruch 3 oder 4, dadurch gekennzeichnet, daß sie wenigstens einen oder mehrere, üblicherweise in der Kosmetik verwendeten/verwendete Hilfsstoff/e enthält, ausgewählt unter Parfüms, Farbstoffen, Konservierungsstoffen, Verdickungsmitteln, anionischen, nichtionischen oder amphoteren Tensiden oder deren Mischungen, Sequestriermitteln, Schaumstabilisatoren, Feuchthaltemitteln, Sonnenfiltern, kosmetisch und dermatologisch aktiven Substanzen.

**6.** Zusammensetzung nach einem der Ansprüche 3 bis 5, bestimmt zur Verwendung als Shampoo, dadurch gekennzeichnet, daß sie zusätzlich ein oder mehrere anionische, nichtionische, amphotere oberflächenaktive Mittel oder deren Mischungen in Anteilen zwischen 0,5 und 30 Gew.-%, bezogen auf das Gesamtgewicht der Zusammensetzung, enthält.

7. Zusammensetzung nach einem der Ansprüche 3 bis 5, bestimmt zur Verwendung als Spülmittel, dadurch gekennzeichnet, daß sie in Form von wässriger Dispersion, Lotion, verdickter Lotion oder Gel vorliegt.

8. Zusammensetzung nach Anspruch 7, dadurch gekennzeichnet, daß sie 1 bis 70 % Lösungsmittel, augewählt unter niederen Alkanolen, Polyalkoholen, Glykolethern und Fettsäureestern, enthält.

9. Zusammensetzung nach einem der Ansprüche 6 bis 8, dadurch gekennzeichnet, daß sie mit einem Mittel verdickt oder geliert ist, welches ausgewählt ist unter: Natriumalginat, Gummi arabicum, Cellulosederivaten, Guargum, Heterobiopolysacchariden, vernetzten oder nicht-vernetzten Acrylsäurepolymeren, einer Mischung von Polyethylenglykol und Polyethylenglykol-stearat oder -distearat, einer Mischung von Phosphorester und Amid, dem Produkt der ionischen Wechselwirkung zwischen einem kationischen Polymeren, das durch ein Cellulosepolymeres oder ein mit einem monomeren, wasserlöslichen quaternären Ammoniumsalz gepfropftes Cellulosederivat gebildet wird, und einem anionischen Carboxylpolymeren, das eine absolute Kapillarviskosität in Dimethylformamid oder Methanol bei einer Konzentration von 5 % und bei 30° C unterhalb oder gleich 30 x $10^{-3}$ Pa.s hat, wobei dieses Verdickungsmittel in Anteilen zwischen 0,1 und 30 Gew.-%, bezogen auf das Gesamtgewicht der Zusammensetzung, vorliegt.

10. Zusammensetzung nach einem der Ansprüche 3 bis 5, bestimmt zur Verwendung als Friseurprodukt ohne Anwendung einer Spülung, dadurch gekennzeichnet, daß sie in wässrigem Medium oder Lösungsmittelmedium die Diorganopolysiloxane mit $\beta$-Ketoesterfunktion, wie in Anspruch 1 oder 2 definiert, enthält, wobei die Lösungsmittel unter niederen $C_2$-$C_4$-Alkanolen und flüchtigen Silikonen ausgewählt sind.

11. Zusammensetzung nach Anspruch 10, dadurch gekennzeichnet, daß sie Verdickungsmittel, ausgewählt unter vernetzten oder nicht-vernetzten Acrylsäurepolymeren, Cellulosederivaten, Ethylen/Maleinsäureanhydrid-copolymeren, Methylvinylether/ Maleinsäureanhydrid-copolymeren, in Anteilen zwischen 0,05 und 5 Gew.-% enthält.

12. Zusammensetzung nach einem der Ansprüche 3 bis 11, dadurch gekennzeichnet, daß sie als Aerosol konditioniert ist, um im Zeitpunkt des Ausstoßens einen Spray in Anwesenheit eines Treibgases zu bilden.

13. Verwendung der Zusammensetzung, wie in einem der Ansprüche 3 bis 12 definiert, bei der kosmetischen Behandlung von Haaren.

14. Verwendung der Zusammensetzung nach einem der Ansprüche 3 bis 5 als Bad- oder Duschprodukt, Körperöl, Bräunungsmittel, Rasierprodukt, parfümierte Lotion, Creme oder Milch.

15. Verfahren zur kosmetischen Behandlung von Haaren zu Verbesserung der Brillanz, der Weichheit, ohne diese zu fetten, dadurch gekennzeichnet, daß man auf diese wenigstens eine Zusammensetzung, wie in einem der Ansprüche 3 bis 12 definiert, aufträgt.

16. Verfahren zur kosmetischen Behandlung der Haut, dadurch gekennzeichnet, daß man auf diese wenigstens eine Zusammensetzung, wie in einem der Ansprüche 3 bis 5 definiert, aufträgt.